# EUROPÄISCHE PATENTSCHRIFT

(11) **EP 1 035 812 B1**
(45) Veröffentlichungstag und Bekanntmachung des Hinweises auf die Patenterteilung: **03.08.2005**
(21) Anmeldenummer: 98950015.2
(22) Anmeldetag: 09.09.1998
(51) Int. Cl.: A61F 2/52

(54) **KIT ZUR HERSTELLUNG EINER BRUSTEPITHESE**
KIT FOR MANUFACTURING A BREAST EPITHESIS
DES BLOC-ELEMENTS POUR LA FABRICATION D'UNE EPITHESE DE SEIN

(30) Priorität: 09.09.1997 DE 19739372; 21.08.1998 DE 19837997
(43) Veröffentlichungstag der Anmeldung: 20.09.2000
(73) Patentinhaber: augmentec Aktiengesellschaft Implantologie-Epithetik, 91799 Langenaltheim (DE)
(72) Erfinder: GEHL, Gerolf, CH-8700 Küsnacht (CH)
(74) Vertreter: Thiel, Christian, Dr. Dipl.-Chem.
(86) Internationale Anmeldenummer: PCT/EP1998/005756
(87) Internationale Veröffentlichungsnummer: WO 1999/012500

(56) Entgegenhaltungen:
- EP-A- 0 617 936
- WO-A-96/00048
- GB-A- 2 243 324
- US-A- 4 401 492
- US-A- 5 458 635

## Beschreibung

Die Erfindung betrifft ein Kit zur Herstellung einer Brustepithese.

Epithesen zum Ausgleich von angeborenen oder erworbenen Körperdefekten sind seit langem bekannt und vielfach beschrieben. Sie werden vielfach eingesetzt, um die Folgen von Unfällen oder chirurgischen Eingriffen zu verdecken und eine natürliche Körperoberfläche vorzuspiegeln. Ein häufiger Einsatzweck ist die Nachbildung der weiblichen Brust nach der chirurgischen Entfernung, bedingt durch bösartige Tumoren.

Epithesen dienen dabei zum einen der Abdeckung der defekten Körperoberfläche gegen unerwünschte äußere Einflüsse, also auch beispielsweise zur Unterstützung der Wundheilung und zur Verbesserung der Hygiene. Zum andern werden Epithesen aber vor allem aus kosmetischen Gründen verschrieben und getragen.

In Anbetracht des Verwendungszwecks ist es erforderlich, die Epithese optimal, an die Körperoberfläche anzupassen. Dies deshalb, weil nur auf diese Art und Weise die medizinischen und hygienischen Rahmenbedingungen erfüllt werden können und weil nur auf diese Weise der erforderliche "Tragekomfort" gewährleistet werden kann. Hinzu kommt, daß nur eine gut sitzende Epithese auch den erwünschten optischen Eindruck erweckt und es dem Patienten erlaubt, sich damit ungezwungen zu bewegen. Eine optimale Nachbildung der gewünschten Körperoberfläche ist dabei selbstverständlich, einschließlich der Nachbildung der natürlichen Hautfarbe.

Herkömmliche Epithesen bestehen aus einer äußeren Hülle, die an die Körperoberfläche angepaßt ist und mit einer Flüssigkeit gefüllt ist. Das Hüllmaterial besteht zumeist aus Siliconkautschuk oder auch einem Polyurethankunststoff, die sich als außergewöhnlich hautfreundlich erwiesen haben. Die Füllung besteht häufig aus einem flüssigen Silicon.

Nachteilig hat sich bei diesen bekannten Epithesen aber die Fließfähigkeit und geringe Formstabilität der Füllung erwiesen. Dieses erfordert besondere Maßnahmen bei der Befestigung der Epithese, die dadurch aufwendiger gestaltet sein muß, als normalerweise als angenehm empfunden wird. Dies, in Verbindung mit dem relativ hohen Gewicht, führt zu Beeinträchtigungen bei der Beweglichkeit und, bei stärkerer Bewegung, auch zum leichten Verrutschen der Epithese. Ferner besteht die Gefahr des Auslaufens bei Verletzung der Hülle. Luftgefüllte Epithesen fallen bei Verletzung der Hülle in sich zusammen.

Nachteilig bei bekannten Epithesen ist ferner ihre geringe Individualität. Konfektionierte Ware ist nur in seltenen Fällen in der Lage, sich an die Besonderheiten des Einzelfalles anzupassen. Eine optimale Anpassung an die Körperoberfläche und das Erscheinungsbild des Trägers ist aber wesentlich für Tragekomfort und Akzeptanz.

Der Erfindung liegt nun die Aufgabe zugrunde, Mittel bereitzustellen, die die Herstellung einer die oben geschilderten Nachteile vermeidenden Brustepithese vereinfachen, wobei diese Brustepithese dennoch in medizinischer Hinsicht unbedenklich ist, hautfreundlich ist - für den Fall des Durchtritts der Füllung durch die Hülle oder der Verletzung der Hülle - und die Elastizität und Formstabilität/Formbarkeit der natürlichen Hautoberfläche besitzen soll. Des weiteren soll die Epithese über lange Zeiträume formstabil bleiben. Insbesondere soll eine individuelle Anpassung der Epithese an die körperlichen Vorgaben des Trägers ermöglicht werden.

Die WO96/00048A beschreibt eine individuell angepaßte Brustepithese und ein Verfahren zu deren Herstellung, bei dem ein Kit gemäß dem Oberbegriff des Anspruchs 1 verwendet werden kann.

Diese Aufgabe wird mit einem Kit gemäss dem Anspruch 1 gelöst.

Mit dem erfindungsgemäßen Kit hergestellte Brustepithesen bestehen aus einer Hülle und einer Füllung. Die Hülle besteht aus einem Kunststoffmaterial, das mit Hilfe einer zweiteiligen Form unter Verwendung eines Körperabdrucks gefertigt ist, der die genaue Anpassung der Epithesenrückseite an den Körper des Trägers erlaubt. Die dabei gefertigte Hülle weist wenigstens eine zum Befüllen mit dem Füllmaterial erforderliche Öffnung auf, die mit einem Stopfen verschlossen ist.

Zur Fertigung der Hülle wird eine individuell gestaltete Schablone verwandt werden, die nach Art eines Abdrucklöffels mit einer Abdruckmasse, etwa einem Alginat, gefüllt wird und verwandt wird, die Kontur des rückseitigen Teils der Form von der Körperoberfläche des Trägers zu nehmen. Die Schablone selbst bildet den äußeren oder Sichtteil der Epithese ab. Der Abdruck von der Körperoberfläche kann auf eine Kunststoffolie übertragen werden, die dann den zweiten, rückseitigen Teil der Form darstellt. Als Materialien für die Form kommen Kunststoffe in Frage, insbesondere Polyester, beispielsweise PET.

Für die Fertigung der Hülle wird die Form insbesondere als Negativform verwandt. Dazu wird, nach der Abnahme des Abdrucks, die in der Form befindliche Abdruckmasse entfernt und werden die beiden Teile der Form miteinander verbunden. In der Form befindet sich zweckmäßigerweise wenigstens eine Öffnung, die auch auf die in der Form auszubildende Hülle übertragen wird. Diese Öffnung ist zum einen zum Befüllen der Form mit der Masse für die Hülle wie auch zum Befüllen der später gebildeten Hülle mit dem Füllmaterial der Epithese notwendig. Bei Brustepithesen befindet sich diese Öffnung zweckmäßigerweise an der Stelle der Mamille.

Zur Ausbildung der Hülle wird das Innere der Form mit einem aushärtbaren Kunststoffmaterial beschichtet, beispielsweise in einer Stärke von etwa 1 mm, das dann zu der Hülle aushärtet. Als Material kommen insbesondere Silicon und Polyurethan in Frage. Ferner können tiefziehfähige Kunststoffolien verwandt werden, die in die Form eingelegt und eingezogen werden.

Sofern ein Aushärten der Hülle erforderlich ist, erfolgt dieses in der Form, um Verformungen zu vermeiden. Das Aushärten kann auf übliche Art und Weise erfolgen, beispielsweise durch Hitzeeinwirkung, chemisch oder durch geeignete Strahlung.

Nach der Fertigstellung der Hülle wird die Hülle in der Form mit dem jeweils gewünschten Füllmaterial befüllt. Das Befüllen in der Form beugt etwaigen Verformungen vor. Als Füllmaterialien kommen in erster Linie Schaummassen und Gele in Frage. Anschließend wird die Hülle verschlossen und ausgeformt.

Es versteht sich, daß die zur Ausbildung der Hülle verwandten Kunststoffmassen entsprechend den Erfordernissen eingefärbt sind.

Von der zweiteiligen Form, die zur Fertigung der Epithese eingesetzt wird, kann der vordere Teil erneut verwandt werden.

Besonders bevorzugtes Material für die Befüllung der Hülle sind Kunststoffschäume, beispielsweise Schaum aus Siliconkunststoff oder Polyurethan. Vorzugsweise wird für den Schaum das gleiche Material verwandt, das auch zur Ausbildung der Hülle eingesetzt wird. Auf diese Art und Weise kann eine Vernetzung der Schaumfüllung mit der Hülle erreicht werden. Schaumgefüllte Epithesen sind in der EP 0 815 811 A beschrieben

Wesentlich ist, daß die Füllung elastisch eingestellt ist, d. h. auf Druck bis zu einem gewissen Grade in sich nachgeben kann, ohne daß dies zum Verrutschen der Epithese führt.

Als geeignet haben sich insbesondere auch Gele gezeigt, die in die Hülle eingebracht werden und bei Druck langsam nachgeben und durch seitliche Verlagerung einen Teil des Drucks zur Seite hin ableiten, danach aber, je nach Einstellung, mehr oder weniger schnell in den Normalzustand zurückkehren. Besonders geeignet ist daher auch eine Füllung, die aus einer wäßrigen Gelatinelösung besteht. Eine solche Füllung ist unter Druck nachgiebig. Besonders geeignet sind ferner Silicongele.

Es versteht sich, daß auch andere übliche Gele bildende Materialien eingesetzt werden, beispielsweise auf Basis wäßriger Lösungen oder Dispersionen von Kieseisäure, Montmorilloniten, Bentoniten, Polysacchariden, Pektinen und dgl.

Besonders bevorzugt sind ferner Lyogele, die sich unter Druck oder Temperatur verflüssigen lassen, danach sich aber wieder verfestigen. Dies ist sowohl für die Herstellung der Epithesen wie auch ihre Performance von Vorteil.

Das Hüllmaterial besteht insbesondere aus einem hautfreundlichen, medizinisch getesteten Siliconkautschuk, der einfärbbar ist und kommerziell erhältlich ist. Typischerweise hat der Kautschuk eine Härte von Shore 20 A. Ein brauchbares Material wird beispielsweise von der Fa. Orthomax, United Kingdom, vertrieben. Geeignet sind ferner Polyurethankunststoffe und Polyethylenterephthalat, wie es beispielsweise von der Firma Erkodent, Mannheim / DE, unter der Bezeichnung Erkodur und Erkoflex vertrieben wird.

Die mit dem erfindungsgemäßen Kit hergestellten Brustepithesen weisen eine Schaumfüllung auf, welche ihnen bei einem geringen Gewicht Formstabilität und zugleich Flexibilität und Rückkehrvermögen in die ursprüngliche Form verleiht. Da keine flüssige Füllung vorhanden ist, ist ein Auslaufen nicht möglich, wie es beispielsweise als Folge von langjährigem Gebrauch oder durch mechanische Beanspruchung bei herkömmlichen Epithesen möglich ist; ebensowenig kann ein Zusammenfallen eintreten, wie bei den bekannten luftgefüllten "Schwimmprothesen". Für mit Gel gefüllte Epithesen gilt bei einem vertretbaren Gewicht entsprechendes; die Auslaufneigung ist durch Verwendung des Gels stark herabgesetzt.

Die mit dem erfindungsgemäßen Kit hergestellten Brustepithesen werden in erster Linie für die Korrektur des Erscheinungsbildes der weiblichen Brust nach Operationen eingesetzt. Sie können aber auch an anderen Körperteilen Verwendung finden, beispielsweise im Bereich des Gesäßes, der Taille oder der Waden. Brustepithesen werden zweckmäßigerweise in den BH, das Mieder oder den Badeanzug eingearbeitet, wodurch der richtige Sitz gewährleistet ist.

Das erfindungsgemäße Kit enthält schließlich eine oder mehrere konfektionierte oder individuell gestaltete Schablonen zur Fertigung der an ein Körperteil angepaßten Hüllen aus einem Kunststoffmaterial zusammen mit einer für die Hüllen geeigneten Kunststoffmasse und Material für die Füllung enthält.

Die Schablonen, die die entsprechende Brust zweckmäßigerweise in mehreren Ausführungsformen nachbilden, sind noch nicht angepaßt. Das jeweils zur Herstellung der Epithese gewählte Exemplar wird zweckmäßigerweise zunächst in Wachs oder Kunststoff dubliert, angepaßt und anschließend in eine Negativform, beispielsweise aus Gips, abgebildet. Aus der Negativform wird dann mit dem jeweiligen Kunststoffmaterial die Epithesenhülle bzw., insbesondere bei Nase, Ohren und Augen, die gesamte Epithese aus dem Kunststoffmaterial ab- bzw. nachgebildet.

Entsprechend könnten auch Fingergliedepithesen konfektioniert und/oder individuell angepaßt werden, wobei als Material vorzugsweise PET verwandt wird, das sich hervorragend reinigen läßt. Fingerepithesen können auch auf einen im Stumpf verankerten Stift aufgesetzt werden, was neben guter Anpassung eine gute Beweglichkeit und Greifsicherheit gewährleistet.

Bei dem erfindungsgemäßen Kit für Brustepithesen stellen die Schablonen beispielsweise Standardmodelle entsprechend gängiger BH-Größen dar, beispielsweise in den Größen A bis D.

Die Schablonen sind als Abdrucklöffel ausgebildet, die dazu verwandt werden, die Kontur der Körperoberfläche, an die die Epithese zu liegen kommt, abzubilden. Dazu enthält das Kit eine übliche Abdruckmasse, beispielsweise auf Alginatbasis. Der Abdruck wird dann dazu verwandt, aus einer Kunststoffmasse oder -folie die Körperoberfläche nachzubilden. Nach Entfernung der Abdruckmasse werden Schablone und verformte Folie zur Form verbunden, die dann zur Ausbildung der Epithesenhülle verwandt wird. Dazu weist die Schablone zweckmäßigerweise eine Öffnung an der Stelle der Mamille auf durch die das Forminnere mit einem aushärtbaren oder einziehbaren Kunststoff beschichtet werden kann. Nach der Aushärtung bzw. Einformung der Hülle wird dann das Hülleninnere, noch in der Form, mit dem Füllmaterial gefüllt und anschließend versiegelt.

Die Versiegelung erfolgt zweckmäßigerweise durch Einkleben der Mamille. Die Schablone dient hier zugleich als Positiv- und Negativform.

Neben den vorstehend genannten Teilen kann das erfindungsgemäße Kit zusätzlich Pigmente und Farbskalen zur Erzielung einer hautgerechten Tönung der Epithese enthalten. Zweckmäßigerweise ist das für die Hülle verwandte Kunststoffmaterial bereits vorgefärbt, so daß nur noch eine Vertiefung bzw. Aufhellung der Grundtönung vorgenommen werden muß. Vorzugsweise enthält das Kit auch Nachbildungen von Mamillen, die auf die fertige Hülle, ggf. auch zum Verschließen der Füllöffnung, aufgeklebt werden. Auch hier kann eine Nachpigmentierung erfolgen.

Die im erfindungsgemäßen Kit zum Einsatz kommenden Schablonen werden in der Regel nach idealisierten Modellen gefertigt und liegen, wie schon erwähnt, zweckmäßigerweise in mehreren Größen vor. Die offene Rückseite solcher Schablonen erlaubt die individuelle Anpassung der Form an den jeweiligen Träger mit Hilfe einer Abdruckmasse. Die Schablone dient somit als Abformlöffel, der mit dem genommenen Abdruck dann in einem zentralen oder angeschlossenen Labor in die fertige Epithese umgesetzt werden kann. Das Kit erlaubt somit die individuelle Anpassung der damit gefertigten Epithese an den Träger und gewährleistet für die damit erstellten Brustepithesen einen optimalen Sitz und Tragekomfort.

## Patentansprüche

1. Kit zur Herstellung einer Brustepithese durch
Fertigen einer Hülle aus einem Kunststoffmaterial mittels Tiefziehen anhand einer zweiteiligen Form unter Verwendung eines Körperabdrucks, wobei die Hülle eine zum Befüllen erforderliche Öffnung im Mamillenbereich aufweist,
Ausschäumen der Hülle,
Verschließen der Hülle und
anschließende Ausformung,
**dadurch gekennzeichnet, daß** es eine oder mehrere individuell gestaltete Schablonen, wobei diese als Abdrucklöffel ausgebildet sind, zur Fertigung der an eine Brust angepassten Hüllen zusammen mit dem Kunststoffmaterial für die Hüllen und Kunststoffmasse für die Füllung enthält.

2. Kit nach Anspruch 1, **dadurch gekennzeichnet, daß** das Kit eine Abdruckmasse sowie eine dauerhaft verformbare Kunststofffolie enthält.

3. Kit nach einem der vorstehenden Ansprüche, **dadurch gekennzeichnet, daß** es Pigmente und Farbskala enthält.

4. Kit nach einem der vorstehenden Ansprüche, **dadurch gekennzeichnet, daß** es Mamillennachbildungen enthält, die zum Verschließen der Füllungsöffnungen geeignet sind.

5. Kit nach einem der vorstehenden Ansprüche, **dadurch gekennzeichnet, daß** die Kunststoffmasse ein Silikon- oder Polyurethankunststoff ist.

6. Kit nach einem der vorstehenden Ansprüche, **dadurch gekennzeichnet, daß** als Kunststoffmaterial Polyethylenterephthalatfolie verwandt wird.

## Claims

1. A kit for the manufacture of a breast epithesis by
forming a casing from a plastic material by deep-drawing using a two-part mould formed from an impression taken from a body, said casing having an opening for filling thereof in the papillar region,
foaming-in of the casing,
sealing the casing and
subsequently demolding,
**characterized in that** it comprises one or more individually designed templates for the manufacturing of the casings adapted to a breast together with the plastic material for the casings and plastic material for the filling, said templates having the form of impression trays.

2. Kit according to claim 1, **characterized in that** kit comprises an impression composition and a permanently deformable plastic foil.

3. Kit according to one of the preceding claims, **characterized in that** it contains pigments and a colour scale.

4. Kit according to one of the preceding claims, **characterized in that** it comprises papillar epithesis members, which are suitable for sealing the filling opening.

5. Kit according to one the preceding claims, **characterized in that** the plastic material is a silicone or polyurethane plastic material.

6. Kit according to one of the preceding claims, **characterized in that** it makes use of a polyethylene terephthalate foil as plastic material.

## Revendications

1. Kit pour la fabrication d'une épithèse de sein par
fabrication d'une enveloppe en matière plastique par emboutissage profond à l'aide d'un moule en deux parties en utilisant une empreinte du corps, l'enveloppe présentant une ouverture nécessaire au remplissage au niveau du mamelon,
moussage de l'enveloppe,
fermeture de l'enveloppe, et
formage final,
**caractérisé en ce qu'**il contient un ou plusieurs gabarits de forme individuelle, configurés en creux d'empreinte, pour la fabrication des enveloppes adaptées à une poitrine, ainsi que la matière plastique pour l'enveloppe et la masse de matière plastique pour le remplissage.

2. Kit selon la revendication 1, **caractérisé en ce que** le kit contient une masse d'empreinte ainsi qu'une feuille plastique déformable durablement.

3. Kit selon l'une des revendications précédentes, **caractérisé en ce qu'**il contient des pigments et une échelle de couleurs.

4. Kit selon l'une des revendications précédentes, **caractérisé en ce qu'**il contient des reproductions de mamelons qui conviennent pour fermer les ouvertures de remplissage.

5. Kit selon l'une des revendications précédentes, **caractérisé en ce que** la masse de matière plastique est une matière plastique à base de silicone ou de polyuréthanne.

6. Kit selon l'une des revendications précédentes, **caractérisé en ce que** l'on utilise comme matière plastique une feuille de polyéthylène téréphtalate.
